# EUROPEAN PATENT APPLICATION

(11) **EP 1 217 070 A1**
(43) Date of publication of application: **26.06.2002**
(21) Application number: 00128136.9
(22) Date of filing: 22.12.2000
(51) Int. Cl.: C12N 15/62, C12N 15/26, C07K 14/55, C07K 14/515, A61K 38/20, A61K 48/00

(54) **Selective cytotoxic fusion proteins**

(71) Applicant: CellGenix Technologie Transfer GmbH, 79110 Freiburg (DE)
(72) Inventor: Cao, Ou, 79106 Freiburg (DE); Kulmburg, Peter, F-68128 Rosenau (FR); Rosenthal, Felicia, D-79102 Freiburg (DE)
(74) Representative: Keller, Günter, Dr.

(57) **Abstract**

The present invention relates to a polynucleotide encoding an immunotoxin for expression in eukaryotic cells. Cells transfected with the polynucleotide secrete the immunotoxin thus having an immunosuppressive effect. The invention also relates to an immunotoxin comprising human interleukin-2 and human angiogenin.

## Description

Hematopoietic growth factors have been shown to accelerate bone marrow recovery after chemotherapy and radiation therapy in mice. In humans, granulocyte colony-stimulating factor (G-CSF) and granulocyte-macrophage colony-stimulating factor (GM-CSF) are widely used to shorten the phase of neutropenia after cytotoxic therapy and after bone marrow transplantation. However, serum half life of these cytokines is relatively short (e.g. 35 minutes for GM-CSF after intraperitoneal injection in mice), and continuous parenteral administration has been used to maintain biologically effective serum concentrations.

Neutropenia after cytotoxic therapy is only transient, and thus, requires cytokine substitution for a limited time period only. It has been shown that clonally selected, human G-CSF gene transfected, irradiated murine fibroblasts can facilitate the release of committed hematopoietic progenitor cells into the circulation and shorten the nadir of drug-induced granulocytopenia in syngeneic mice (Rosenthal et al. (1996) Hum. Gene Ther. 7, 2147-2156). G-CSF plasmid vector has also been transfected into allogeneic fibroblasts and it has been found that the biological effect of one single application of irradiated human G-CSF expressing murine allogeneic fibroblasts is identical or even better on neutropenia recovery than daily applications of a much higher dose of the recombinant cytokine. However, this effect cannot be reproduced in a second cycle of chemotherapy followed by the transplantation of allogeneic gene transfected cells. This lack of reproducibility most likely is due to an immune response against the gene transfected cells which is mediated primarily by T cells.

In keeping with the primary roles of T cells in graft rejection, most immunosuppressive drugs currently in use or under development target T cells. The most successful drugs act in the early phase of T cell activation. For example, cyclosporine and tacrolimus (formerly known as FK 506) each prevent T cells from producing cytokines critical for T cell proliferation, such as interleukin-2. Sirolimus (rapamycin), a promising drug in phase 3 clinical trials, blocks signal transduction by the interleukin-2 receptor. Azathioprine and mycophenolate, both inhibitors of purine synthesis, also inhibit T cell proliferation. Monoclonal antibodies directed at T cells, such as OKT3 (anti-CD3), act by depleting T cells and blocking the T cell antigen receptor. Although these drugs have led to considerable improvement in survival rates for both patients and grafts, nonspecific immunosuppression is the major problem associated with their use. Patients taking them have a substantial risk of opportunistic infections and of developing cancer. There are also side effects not associated with their immunosuppressive actions, such as the nephrotoxic effects of cyclosporine and tacrolimus and the diabetogenic effects of tacrolimus.

One objective of the invention therefore is to provide an advantageous immunosuppressive agent or composition.

Surprisingly, it has been found that transfected eukaryotic cells secreting an immunotoxin prevent the killing of allogeneic cells by activated T cells in vivo. After administration to mice, the transfected cells continue to secrete the immunotoxin in vivo. The secreted immunotoxin is a fusion protein which comprises a polypeptide binding to a receptor present on T cells and a polypeptide which is toxic to cells upon internalization. The immunotoxin via its cell surface receptor binding portion binds to the cells and is internalized. The toxin portion subsequently leads to killing of the targeted cell.

The invention therefore relates to a polynucleotide comprising a nucleotide sequence capable of acting as a eukaryotic promoter or a nucleotide sequence capable of acting as a viral promoter, and a nucleotide sequence encoding a fusion protein which comprises a first polypeptide capable of binding to a cell surface receptor present on human cells and a second polypeptide which is toxic to a human cell after internalization of said second polypeptide by the human cell. Usually the polynucleotide further comprises a nucleotide sequence capable of acting as a polyadenylation signal. Optionally, the polynucleotide further comprises a nucleotide sequence encoding an amino acid sequence which is capable of acting as a secretion signal in eukaryotic cells.

The polynucleotide of the invention may comprise any nucleotide sequence which is capable of acting as a eukaryotic promoter or any nucleotide which is capable of acting as a viral promoter. Many promoter nucleotide sequences regulating the expression of genes in eukaryotic and/or mammalian cells are known in the art. In a preferred embodiment the nucleotide sequence capable of acting as a eukaryotic promoter is the promoter nucleotide sequence of elongation factor 1α (EF1α).

The polynucleotide of the invention preferably is an expression vector suitable for expressing genes in mammalian cells. The polynucleotide usually is a circular polynucleotide. It may further comprise a replication origin allowing the amplification of the polynucleotide in bacteria, and a nucleotide sequence encoding a polypeptide conferring resistance against a substance which inhibits the growth of bacteria. Substances which inhibit the growth of bacteria are many antibiotics such as ampicillin or kanamycin. The polynucleotide of the invention may contain the ampicillin-resistance gene encoding β-lactamase which inactivates ampicillin. Bacteria successfully transformed with the polynucleotide can be easily selected from the larger number of cells that are not transformed by growing them in an ampicillin-containing medium.

In a preferred embodiment the polynucleotide of the invention further comprises a nucleotide sequence encoding a polypeptide conferring resistance to a substance which inhibits the growth of eukaryotic cells. Such nucleotide sequence can be a "resistance gene" which is widely used to select successfully transfected mammalian cells. Examples of such resistance genes are the genes encoding neomycin phosphotransferase or hygromycin phosphotransferase. These enzymes inactivate neomycin and hygromycin, respectively, which inhibit the growth of mammalian cells not containing said enzymes. In a particular embodiment the nucleotide sequence encoding the fusion protein and the nucleotide sequence encoding the resistance factor for the selection of transfected cells are on the same plasmid. It has been found that positively selected cell clones often fail to express the gene of interest at significant levels when marker and the gene of interest are encoded on separate transcription units. Therefore, dicistronic expression vectors, in which two cDNAs are linked by the internal ribosome entry sequence (IRES) of picorna viruses, are preferred. Generally, the downstream cistron of a dicistronic mRNA is translated much less efficiently than the upstream one. However, if picorna virus 5'-untranslated region (5'-UTR) sequences are inserted between the two cistrons, the protein product of the downstream cistron can even be detected earlier in higher yield than the upstream one. This implies that ribosomes can bind to the picorna viral UTR segment and initiate translation of the downstream cistron without having to translate the upstream cistron first.

The fusion protein encoded by the polynucleotide of the invention comprises a first polypeptide and a second polypeptide. The terms "first polypeptide" and "second polypeptide" do not indicate the sequence of the two polypeptide portions within the fusion protein. Thus, in one embodiment the first polypeptide may be the N-terminal portion and the second polypeptide the C-terminal portion, in another embodiment the second polypeptide may be the N-terminal portion and the first polypeptide the C-terminal portion.

The polynucleotide of the invention comprises a nucleotide sequence encoding a fusion protein which comprises a first polypeptide capable of binding to a cell surface receptor present on human cells. Usually, the cell surface receptor is not present on all human cells but only on a certain population of cells. The first polypeptide is preferably capable of binding to a cell surface receptor which is present on human T cells. More preferably, the cell surface receptor is present on activated T cells and substantially not present on resting T cells and memory T cells. Still more preferably the first polypeptide is capable of binding to a cytokine receptor present on human cells. Most preferably the cytokine receptor is human IL-2 receptor.

The polynucleotide of the invention preferably encodes a fusion protein which comprises as a first polypeptide a cytokine or a fragment thereof capable of binding to a cell surface receptor present on human cells. Still more preferably, the first polypeptide of the fusion protein is a human cytokine or a fragment thereof capable of binding to a cytokine receptor.

According to the invention, cytokines are molecules selected from the group consisting of interleukins, interferons and haematopoietic, epidermal or other cellular growth factors. In the most preferred embodiment the human cytokine is human interleukin-2 (IL-2) (Taniguchi it al. (1983) Nature 302, 305). Generally, the first polypeptide of the fusion protein may also be a fragment of a cytokine, provided that this fragment is still capable of binding to the cell surface receptor. Another cytokine that can be the first polypeptide of the fusion protein is epidermal growth factor (EGF).

The first polypeptide which is capable of binding to a cell surface receptor present on human cells may also be an antibody, an antibody fragment or a scFv fragment. Antibodies and their fragments and derivatives can be raised against a variety of cell surface receptors by methods known in the art. Once the nucleotide sequence encoding the antibody or its derivatives is known, the nucleotide sequence can be incorporated in the polynucleotide of the invention to encode the first polypeptide. The antibody or its derivative may be a "humanized" form to reduce the risk of inducing an immune response.

The fusion protein encoded by the polynucleotide of the invention further comprises a second polypeptide which is toxic to a human cell after internalization of said second polypeptide by the human cell. The second polypeptide may be any toxin, however, it is preferred that it is not *Pseudomonas* exotoxin A or a fragment thereof. It is preferred that the second polypeptide is a ribonuclease or a fragment thereof having ribonuclease activity.

Usually, the second polypeptide of the fusion protein is a ribonuclease of the superfamily of human pancreatic RNases. It is preferred that the second polypeptide of the fusion protein is human angiogenin or a fragment thereof having ribonuclease activity (Kurachi et al. (1985) Biochemistry 24, 5494-5499).

Angiogenin is a human protein with homology to pancreatic ribonuclease and ribonuclease activity albeit different from that of the pancreatic enzyme. The purified protein was shown to have potent activity as an angiogenic factor. Angiogenin is also a potent inhibitor of protein synthesis in cell-free extracts and upon injection into Xenopus oocytes. Extracellular angiogenin is not cytotoxic towards a wide variety of culture cells and is normally present in human plasma.

Preferably, the polynucleotide of the present invention further comprises a nucleotide sequence encoding a spacer linking the first and the second polypeptide of the fusion protein. The spacer usually consists of 1 to 50 amino acids. The spacer sequence in the fusion protein provides some flexibility of the two polypeptide portions with respect to each other. Preferably, the nucleotide sequence encoding the spacer is a nucleotide sequence encoding 3 to 20 amino acids. It is also preferred that the nucleotide sequence encoding the spacer of the fusion protein encodes only the amino acids serine and glycine. Most preferably, the nucleotide sequence encoding the spacer encodes the amino acid sequence (GGGGS)₃ (SEQ ID NO:1).

It is clear to the skilled person that due to the degeneracy of the genetic code a specific fusion protein may be encoded by many different polynucleotides. The skilled person is also aware of the fact that only minor substitutions in the amino acid sequence of the fusion protein which result in amino acids which are different from the amino acids present in the respective native human protein are possible without destroying the biological function of the polypeptide and/or without substantially changing the immunogenicity of the fusion protein. Therefore, such substitutions in the amino acid sequences of the first or the second polypeptide which result in sequences which are different from the native sequences are also encompassed by the present invention.

The polynucleotides of the present invention may be used to transfect eukaryotic cells, preferably mammalian cells which may then further be used as immunosuppressive agents in vivo.

Another aspect of the invention is a eukaryotic cell, preferably a mammalian cell transfected with a polynucleotide of the present invention. More preferably, the eukaryotic cell is a human fibroblast cell, most preferably the eukaryotic cell is a human skin fibroblast cell. Skin fibroblasts have been found to be particularly attractive because of their accessibility by biopsy, their comparatively long life span and short population doubling time prior to senescence in vitro, and their relatively high transfection efficiency and transgene expression. In one embodiment the eukaryotic cells of the invention are not derived from immortalized cell lines. Preferably, the eukaryotic cells are allogeneic cells. In other embodiments the eukaryotic cells may also be autologous cells. The cells may be irradiated before or after transfection. The irradiated cells have lost the capability to proliferate in vivo, but have retained the ability to secrete biologically active levels of immunotoxin for several days. Thus, the time period of secretion is limited. Moreover, irradiation is a precaution when cells are injected in vivo to exclude malignant transformation and proliferation of transfected cells after in vivo implantation. The preferred irradiation dose is 50 to 100 Gy.

The eukaryotic cells of the invention can be administered to patients through different routes. Preferably, they are administered by subcutaneous injection. Other routes might be intraperitoneal or intramuscular. Usually 5×10⁵ to 5×10⁸ cells, preferably 5×10⁶ to 5×10⁷ cells are administered in a volume of 0.1 to 1 ml, preferably in a volume of about 0.5 ml.

The eukaryotic cells of the invention can be administered to patients to suppress the immunological response against G-CSF gene transfected fibroblasts and their transgene products and ultimately induce tolerance. In one embodiment the transfected cells when administered to the patient are capable of killing alloreactive T cells bearing cytokine receptor. The cells expressing the fusion protein are also expected to be useful in other settings where alloreactivity has to be suppressed, even in organ allotransplantation.

Another aspect of the present invention therefore is a pharmaceutical composition containing a eukaryotic cell as described supra. The pharmaceutical composition may further contain a pharmaceutically acceptable diluent or carrier, e.g. a suitable buffer or salt solution.

The invention also concerns a method for the preparation of a pharmaceutical composition wherein a eukaryotic cell is transfected with a polynucleotide of the invention. Preferably the eukaryotic cell to be transfected is a human fibroblast, more preferably the cell is a human skin fibroblast. The eukaryotic cells can be transfected by a method known in the art such as calcium phosphate mediated transfection, electroporation, lipofection, etc. It is preferred, however, that the eukaryotic cells are transfected using a liposomal transfection reagent. The most preferred reagent is "DOSPER" obtainable from Roche Molecular Biochemicals. The transfected cells are selected by culturing them in the presence of an inhibitory substance such as neomycin or hygromycin. Successfully transfected cells are not inhibited when cultured in the presence of the substance. After cloning of individual colonies and expansion of the clones, the different clones can be characterized as to the expression level of the fusion protein and the amount of secreted fusion protein. Isolated clones may also be frozen for later use. Prior to administration to the patient, the cells may be irradiated as described supra.

Excellent results have been obtained using a novel polypeptide comprising IL-2 and angiogenin as a fusion protein. The invention therefore also relates to a polypeptide comprising IL-2 or a fragment thereof which is capable of binding to a human IL-2 receptor, optionally a spacer consisting of 1 to 50 amino acids, and angiogenin or a fragment thereof which has ribonuclease activity. Preferably, the polypeptide comprises human IL-2 and/or human angiogenin.

It is preferred that the polypeptide comprises complete human IL-2 and/or complete human angiogenin. It is preferred that a spacer consisting of 3 to 20 amino acids links the IL-2 portion and the angiogenin portion of the fusion protein. Different spacer compositions can be envisaged, however, spacers consisting of an amino acid sequence which is composed only of the amino acids serine and glycine are preferred. The most preferred spacer has the amino acid sequence (GGGGS)₃(SEQ ID NO:1).

The polypeptide may be purified from prokaryotic or eukaryotic cells upon expression of recombinant DNA encoding the polypeptide. The invention also relates to a polynucleotide encoding a polypeptide comprising IL-2 or a fragment thereof which is capable of binding to human IL-2 receptor, optionally a spacer consisting of 1 to 50 amino acids, and angiogenin or a fragment thereof which has ribonuclease activity. The polynucleotide can be used to express the IL-2/angiogenin fusion protein in bacteria or eukaryotic cells to obtain the fusion protein in purified form.

Another aspect of the invention is the use of a polynucleotide, a eukaryotic cell and/or a polypeptide of the present invention for the preparation of an immunosuppressive medicament. The medicament may be used before, during or after chemotherapy or for the prevention of allograft rejection together with and after transplantation. It may also be useful for the treatment of disorders of the immune system such as hypersensitive conditions.

The present invention provides an advantageous method for the delivery of an immunosuppressive agent or composition to a patient. Administration of transfected cells to the patient guarantees a continuous delivery of the active agent. Irradiation of the transfected cells provides the possibility to limit the time period of secretion, since irradiated cells have lost the capability to proliferate in vivo, but have retained the ability to secrete biologically active levels of immunotoxin for several days. The present invention provides the means for a successful preparation and administration of these cells. Another advantage of the subject matter of one embodiment of the present invention is that the cells to be administered to the patient secrete a fusion protein comprising as two portions two human proteins or fragments thereof. Thus, the likelihood to elicit an immune response in patients against the secreted fusion protein is greatly reduced. The immunotoxins of the invention are furthermore capable of selectively killing activated T cells without exhibiting cytotoxicity against other cells. The disadvantages of other immunosuppressive agents have thus been overcome.

Figure 1 outlines the construction of the AnglL2 eukaryotic expression vector. The abbreviations used are: EF1a, promoter of elongation factor 1α; IL-2, cDNA of human interleukin-2; Ang, cDNA of human angiogenin; IRES, internal ribosome entry sequence; neo, cDNA of neomycin phosphotransferase; polyA, small intron and polyadenylation signal from SV40; NTS, nontranscribed spacer; L, linker. Only restriction sites relevant for cloning are shown.

Figure 2 shows the AngIL2 eukaryotic expression vector. The abbreviations are the same as described for figure 1.

Figure 3 shows a western blot analysis of cell lysates in culture supernatant of untransfected BALB3T3 and KMST6 cells, IL-2 secreting KMST6 cells, AnglL2 transfected BALB3T3 clones and recombinant IL-2. After SDS-PAGE electrophoresis, the gel was transferred to a nitrocellulose membrane and probed with an antibody against IL-2. Bands were visualized by a horseradish peroxidase-labeled secondary antibody and luminescence. Figure 3 represents the result of example 3.

Figure 4 shows a FACS analysis of IL-2 receptor expression level of allogenic activated PBL. For further details see example 4.

Figure 5 shows the result of the XTT assay of example 4. The two days' supernatant of AngIL2/BALB#3 had a similar killing activity as the positive control actinomycin.

Figure 6 shows the absolute leukocyte counts after chemotherapy and therapy with G-CSF gene-transfected cells and AngIL2 gene-transfected cells. Results of example 5 are shown.

The following examples further illustrate the invention.

### Example 1: Construction of eukaryotic expression vector encoding AngIL2

### a) mRNA extraction from NS3 cells

mRNA from NS3 cells (a human endothelial cell line) was isolated using Pharmacia Quick Prep Micro mRNA purification Kit according to the manufacturers instructions. 1x10⁷ NS3 cells were resuspended in 1 ml of PBS and pelleted by centrifugation in a microcentrifuge tube. The supernatant was removed. 0.4 ml of Extraction Buffer was added to the pelleted cells and vortexed until a homogeneous suspension was achieved. The sample was diluted by adding 0.8 ml of Elution Buffer and mixed using a vortex mixer. A cleared cellular homogenate was prepared by centrifuging each extracted sample for 1 min at top speed. The tube containing 1 ml Oligo(dT)-Cellulose was also be centrifuged for 1 min. The buffer from Oligo(dT)-Cellulose pellet was removed and 1ml of the cleared cellular homogenate was placed on the top of the pellet of Oligo(dT)-Cellulose. The mixture was gently mixed for 3 min and centrifuged at a maximum speed for 10 sec. The supernatant was removed. Then the Oligo(dT)-Cellulose was washed with High-Salt Buffer five times followed by washing with Low-Salt Buffer two times. The resin was resuspended in 0.3 ml of Low-Salt Buffer and the slurry was transferred to a MicroSpin Column placed in a microcentrifuge tube. The tube was centrifuged at full speed for 5 sec. The effluent in the collection tube was discarded and the column was washed with 0.5 ml of Low-Salt Buffer for 3 times. Then the column was placed in a microcentrifuge tube. 0.2 ml of Elution Buffer prewarmed to 65°C was added to the top of the resin bed followed by centrifugation at top speed for 5 sec. Elution step was repeated once more and 400 µl of eluate was obtained. mRNA in the eluate was quantified and precipitated.

### b) First strand cDNA synthesis

mRNA extracted from NS3 was reverse-transcribed using the Superscript Preamplification system according to manufacturer instructions. Random primers were used in the reaction. The RNA/primer mixture was prepared in a sterile 0.5 ml tube: 10 µl of mRNA (6.24 µg/ml) was mixed with 2 µl of random hexamers (50 ng/µl) and incubated at 70°C for 10 min followed by incubation on ice for at least 1 min. 2 µl of 10x PCR buffer, 2 µl of 25 mM MgCl₂, 1 µl of 10 mM dNTP mix, and 2 µl of 0.1 M DTT were mixed to prepare a reaction mixture. The reaction mixture was added to RNA/primer mixture and incubated at 25°C for 5 min. 1 µl (200 units) of Superscript II RT was added and mixed. Then the reaction mixture was incubated at 25°C for 10 min, 42°C for 50 min and 70°C for 15 min in a PCR cycler. 1 µl of RNase H was added and incubated at 37°C for 20 min before proceeding to synthesis of hAng-specific cDNA. The product was stored at -20°C.

### c) Human angiogenin specific cDNA synthesis by PCR (1)

Using the human angiogenin specific primers Kbg 379 (SEQ ID NO:2) and Kbg 380 (SEQ ID NO:3), hAng-specific cDNA was amplified by PCR 30 cycles (45 sec at 94°C, 45sec at 55°C and 3 min at 72°C) using Taq DNA Polymerase. The PCR product was a fragment of approximately 400 bp as shown on agarose gel.

Kbg 379 (SEQ ID NO:2):	5'-GGT GCC ACC ATG GTG ATG GGC CTG GGC
GTT TT-3'

Kbg 380 (SEQ ID NO:3):    5'-GCT GCC CCC TCC GCC CGG ACG ACG GAA AAT
TGA CTG-3'

### d) Synthesis of hAng+linker by PCR (2)

The amino acid sequence (GGGGS)₃ (SEQ ID NO:1) was inserted into the AngIL2 fusion protein gene linking the sequence coding for hAng and hIL2. The sequence coding for the linker was introduced by PCR with specific primers.

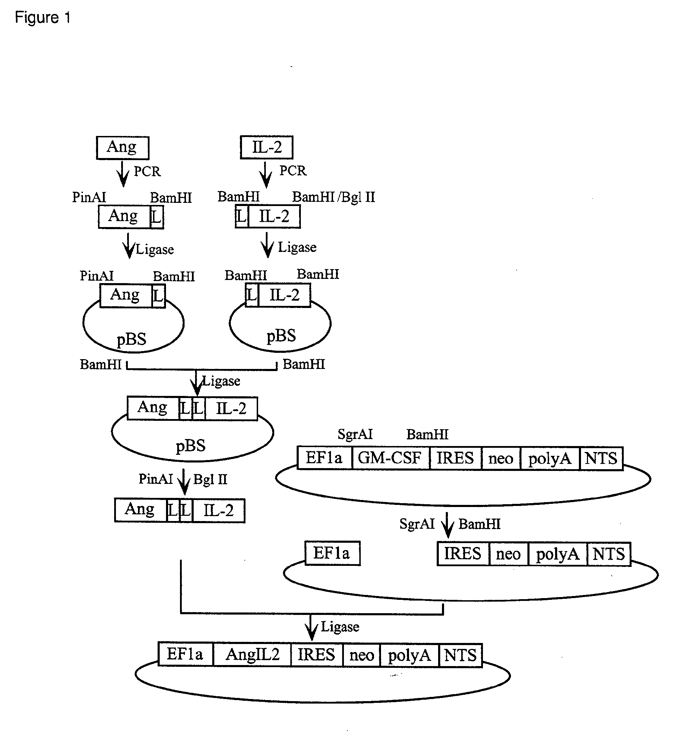

A schematic representation of the cloning strategy is shown in Figure 1.

With primers Kbg 381 (SEQ ID NO:4) and Kbg 382 (SEQ ID NO:5), the sequence coding for hAng+Linker was amplified by PCR using 30 cycles (45 sec at 94°C, 45 sec at 55°C and 3 min at 72°C) with pfu polymerase using hAng PCR (1) product as template. hAng PCR (product) was subcloned into pBluescript after digestion with BamHI and HindIII and sequenced. Ang cDNA plus linker was detected on agarose gel at the expected size of 420 bp.

Kbg 381 (SEQ ID NO:4):     5'-GATC AAG CTT ACC GGT GCC ACC ATG GTG AT-3'

Kbg 382 (SEQ ID NO:5):	5'-CGCC GGA TCC CCC GCC TCC GCT GCC CCC
TCC GCC CGG ACG-3'

### e) Synthesis of hIL2+Linker by PCR

The sequence encoding hlL2 with Linker was amplified by PCR by specific primers Kbg383 (SEQ ID NO:6) and Kbg384 (SEQ ID NO:7) using the same program and polymerase as for hAng PCR (2). The PCR product was subcloned into pBlueScript after digest with BamHI and sequenced. The IL2 PCR product was about 450 bp. The primers used for PCR were:

Kbg 383 (SEQ ID NO:6): 5'-CGGG GGA TCC GGC GGA GGG GGA AGC GCA
CCT ACT TCA AGT TCT ACA-3'

Kbg 384 (SEQ ID NO:7):	5'-GATC GGA TCC AGA TCT TCA AGT TAGTGT TGA
GAT GAT-3'

### f) Fusion of Ang-IL2 in pBlueScript

The pBS plasmids containing hAng+Linker or hIL2+Linker were digested with BamHI. The IL2 fragment was then fused to the pBS carrying hAng at BamHI site. The fusion gene coding for hAng-Linker-IL2 was sequenced.

### g) Construction of expression vector

A dicistonic eukaryotic expression vector was used to express the AnglL2 fusion protein. The plasmid vector originally containing GM-CSF was digested by SgrA/BamHI and linearized. GM-CSF was a fragment of approximate 450 bp as shown on agarose gel and removed from the plasmid vector. The AnglL2 fragment was obtained by digestion with BgIII/PinAl of fusion gene containing Bluescript and separated by gel electrophoresis at the expected size of 900 bp. Then the fusion gene was inserted into the expression vector via ligation.

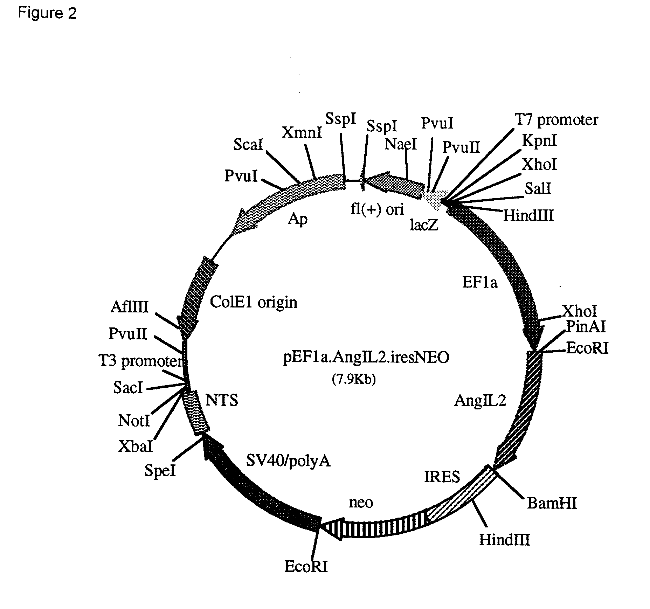

The structure of the final AnglL2 expression vector is shown in Figure 2.

### Example 2: Transfection of AnglL2 in murine fibroblasts BALB3T3

The day before transfection, 1x10⁵ BALB3T3 were seeded in 2 ml culture medium per well of a 6-well plate. The cells were incubated at 37°C in an incubator until 60-80% were confluent. On the day of transfection, different ratios (2:1 and 3:1) of the transfection reagent (DOSPER)/DNA mixture were prepared and incubated at room temperature for 15 min to allow the DOSPER/DNA complex to form. The culture medium was replaced with 1 ml of fresh culture medium shortly before adding the DOSPER/DNA mixture. 100 µl of the DOSPER/DNA complex was added drop by drop across the entire well to each well. The cells were incubated with DOSPER/DNA overnight at 37°C. The day after transfection, the transfection medium was replaced with 2 ml fresh growth medium per well. 48 hours after transfection, the cells were trypsinized and diluted in selective medium (0.5 mg/ml G418).

### Dilution cloning and selection

48 hours after transfection, the cells were trypsinized and diluted 1:5, 1:10 and 1:20 in DMEM medium containing 0.5 mg/ml G418. Then the cells were seeded in 6-cm petridishes and incubated at 37°C for 2-3 weeks. Every 3 days the medium was replaced by fresh medium containing 0.5 mg/ml G418. When colonies were big enough, they were marked on the underside of the dishes. One sterile cloning ring was dipped in sterile silicone grease, pressed down on the dish alongside silicone grease to spread the grease round the base of the ring. The ring was placed around desired colony. It was repeated for two or three other colonies in the same dish. 100 µl of trypsin/EDTA was added to the hole in the ring and left for 20 sec and removed. The dish was closed and incubated for 15 min. Then 100-200 µl of medium was added to each ring and pipetted up and down to disperse cells. Each clone was taken in turn and transferred to a well of a 24-well plate. When clones had grown to fill wells, they were transferred up to a 25 cm² flask and incubated conventionally with 5 ml medium.

### Example 3: Secretion of AngIL2 in transfected BALB3T3

G418-resistant, bulk-transfected and randomly selected clones from transfection were screened for hIL2 and hAng expression by analyzing release of both cytokines into culture supernatants.

### a) Supernatant collection

For cytokine quantitation of transfected cells, the culture supernatant from parental cells and fusion protein transfected clones were collected. For each clone 7.5x10⁵ cells were cultured in one 75 cm² culture flask (1x10⁴ cells per cm²). After 24 hours' incubation, the culture medium were removed and 10 ml fresh medium containing G418 were added. After 24 hours, the medium was collected from the cells and centrifuged at 3500rpm for 10 min at 4°C. 1 ml of supernatant was frozen at -20°C for further use. Meanwhile the cells were trypsinized and counted.

### b) ELISA

Culture supematants from semiconfluent AngIL2 transfected clones and parental cells were assayed for hlL2 and hAng production by human IL2 and human ANG ELISA kits, respectively. The supernatants from untransfected BALB3T3 were used as negative control. The assays were performed according to manufacturers' instructions. Data were calculated as cytokine production per 24 hours and 10⁶ viable cells and means of duplicate determinations.

Untransfected BALB3T3 did not secrete any detectable hIL2 or hAng. Also several neomycin-resistant clones failed to express either hIL2 or hAng at high levels. Table 1 shows IL2 and Ang secretion of BALB3T3 clones with high expression.

**Table 1**

| clone | IL2 | Ang |
|---|---|---|
| | (pg/10⁶ cells and 24hrs) | |
| 3 | 81000 | 87000 |
| 7 | 53000 | 52000 |

### c) Western Blot

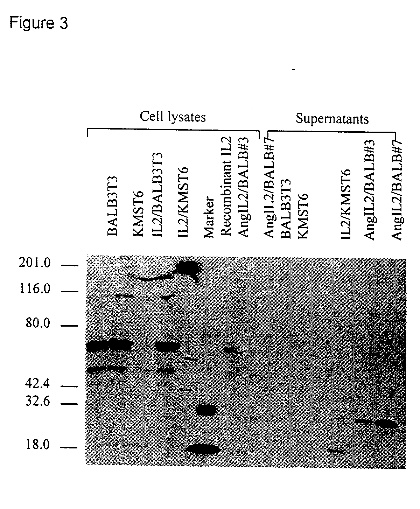

To verify secretion of the fusion protein AnglL2, culture supernatants and cell lysates of AnglL2 transfected clones were analyzed by SDS-PAGE and Western immunoblot (Fig. 3).

In the culture supernatants of transfected BALB clones, an anti-hlL2 mAb detected a protein band with an apparent molecular mass of 28 kDa, whereas no specific signal could be found in the supernatants of the untransfected cells. Several protein bands were detected by IL2 mAb with the apparent molecular mass of 50 and 60 kDa in the cell lysates of both untransfected and transfected BALB3T3 and KMST6. In the supernatant of IL2 transfected KMST6 and in controls containing recombinant human IL2, a protein of molecular mass of 14 kDa was detected by anti-hlL2 mAB. A protein band with molecular mass of 28 kDa was found in control containing recombinant IL2 presumably due to the formation of IL2 dimers. This result shows that AngIL2 is secreted by transfected fibroblasts.

It should also be noted that AngIL2 did not have a cytotoxic effect on the transfected cells.

### Example 4: Biological activity of AngIL2 in vitro

### a) Allogenic specific lymphocyte expansion for bioassay

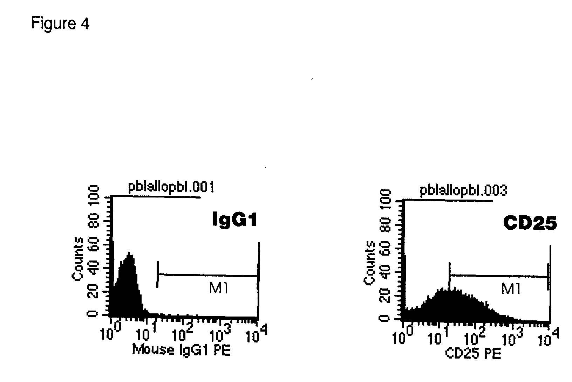

Peripheral blood lymphocyte (PBL) were isolated from two healthy donors using Ficoll separation. 1 x 10⁴ of donor#1 PBL were cultured with 7 x 10³ of donor#2 PBL irradiated for 35 Gy and 3 x 10³ of LAZ388 irradiated for 60 Gy per well of 96-well plate for 7 days at 37°C. On day 7, the cells were harvested and some of them are frozen. The cells were recultured in the same conditions as day 0. On day 10, the cells were harvested and CD25 antigen positive cells are determined by flowcytometer (Fig. 4). Meanwhile these cells were used for bioassay.

### b) Bioassay

Allogenic stimulated PBL were used to test the killing activity of AngIL2 fusion protein. On day 10 of allogenic lymphocytes expansion, 5x10⁴ or 2.5x10⁴ of PBL were cultured per well of 96-well plate (flat bottom) with the supernatants from untransfected BALB3T3 or AngIL2 secreting cells and incubated at 37°C in 5% CO₂ atmosphere. After 2 days, cell proliferation and viability were detected by XTT assay. Cell proliferation kit II (XTT, Roche) were used for the measurement of cytotoxicity of immunotoxins. After the incubation period of cells and supernatant, XTT labeling reagent and electron-coupling reagent were thawed respectively in a waterbath at 37°C. 5 ml of XTT labeling reagent was mixed with 0.1 ml of electron coupling reagent for one microtiter plate (96 wells). 50 µl of the XTT labeling mixture was added to each well and incubated for 4 to 24 hours at 37°C in 5% CO₂ atmosphere. The spectrophotometrical absorbance of the samples were measured at 450nm using the microtiter plate (ELISA) reader at 4, 8, 24 hours after addition of XTT labeling mixture.

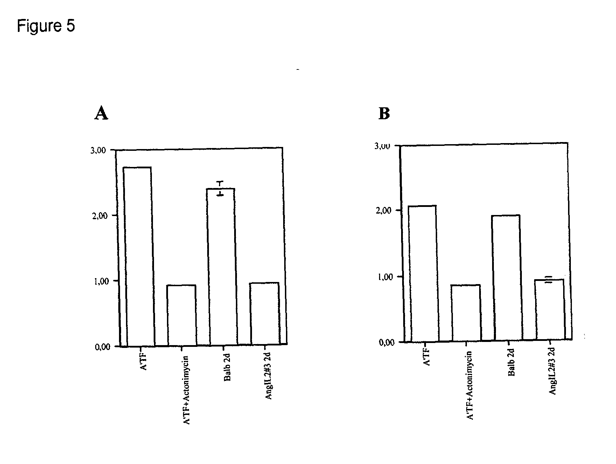

In this XTT proliferation assay, the supernatant of untransfected BALB3T3 was used as negative control and exhibited no any cytotoxic effect on allogeneic activated lymphocytes. The positive control, using actinomycin had significant killing effects on activated lymphocytes. The two days' supernatant of AngIL2/BALB#3 had similar killing activity as actinomycin (Fig. 5).

### Example 5: Protective effect on hG-CSF secreting BALB3T3 in allogenic mouse transplant model

The question of whether AngIL2 transfected fibroblasts could suppress activated T cells was addressed in a repetitive allotransplantation mouse model.

### a) Chemotherapy and transplantation

Female C57BI/6 mice were purchased from Charles River and housed in a temperature-and humidity-controlled environment. Mice were injected intraperitoneally (i.p.) on day 0 and -2 of 1st and 2nd chemotherapy cycles with 150 mg/kg of cyclophosphamide and divided into groups. 30 days before 1st chemotherapy, pretreatment was done by injection s.c. with untransfected or transfected Balb3T3.

**Tab. 2.**

| C57/BI6 mice were divided into 7 groups followed by injection with parental cells or gene transfected BALB3T3. Each group contained 3 mice. | | | |
|---|---|---|---|
| group | pretreatment | 1^{st} cycle | 2^{nd} cycle |
| 1 | -- | -- | -- |
| | | | |
| 2 | G-CSF/BALB | G-CSF/BALB | G-CSF/BALB |
| | | | |
| 3 | BALB/3T3 | G-CSF/BALB | G-CSF/BALB |
| | | | |
| 4 | AngIL2/BALB | G-CSF/BALB AngIL2/BALB | G-CSF/BALB |
| | | | |
| 5 | AngIL2/BALB | G-CSF/BALB | G-CSF/BALB |
| | | | |
| 6 | -- | G-CSF/BALB | G-CSFIBALB |
| | | | |
| 7 | -- | -- | G-CSF/BALB |

The mice in group 1 received neither pretreatment nor transplantation.

Group 2 received pretreatment by injection with 1x10⁷ G-CSF/Balb3T3 and the injection with 1x10⁷ G-CSF/BaIb3T3 per mouse on Day 0 in both chemotherapy cycles.

Group 3 received pretreatment by injection with 1x10⁷ BALB3T3 and the same transplantation on day 0 in both chemotherapy cycles per mouse.

Group 4 received 1x10⁷ AngIL2/BALB3T3 injection as pretreatment and co-transplantation of hG-CSF/BALB3T3 and AngIL2/BALB3T3 on day 0 in the 1st cycle. In the 2nd cycle, group 4 only received the injection with hG-CSF/BALB3T3 on day 0.

Group 5 received 1x10⁷ of AngIL2/BALB3T3 injection as pretreatment and hG-CSF/BALB3T3 transplantation on day 0 in both cycles.

Group 6 received hG-CSF/BALB3T3 transplantation on day 0 in both cycles without pretreatment.

Group 7 received only hG-CSF/BALB3T3 transplantation in the 2nd cycle.

1x10⁷ irradiated hG-CSF/BALB3T3 and/or AngIL2/BALB3T3 cells were injected per mouse in both pretreatment and transplantation of two chemotherapy cycles. The interval between two chemotherapy cycles is 36 days (Tab. 2).

### b) Blood collection and measurement of peripheral blood cells

Peripheral blood were drawn on day -2, 3, 6, 7, 8, 10 in the both chemotherapy cycles. 20 µl peripheral blood per mouse were taken from orbital sinus or plexus of the mice and diluted in 180 µl of Abbott Counter diluent followed by measurement of peripheral blood cells by Abbott Cell Dyn 3500 using program Correct Mouse.

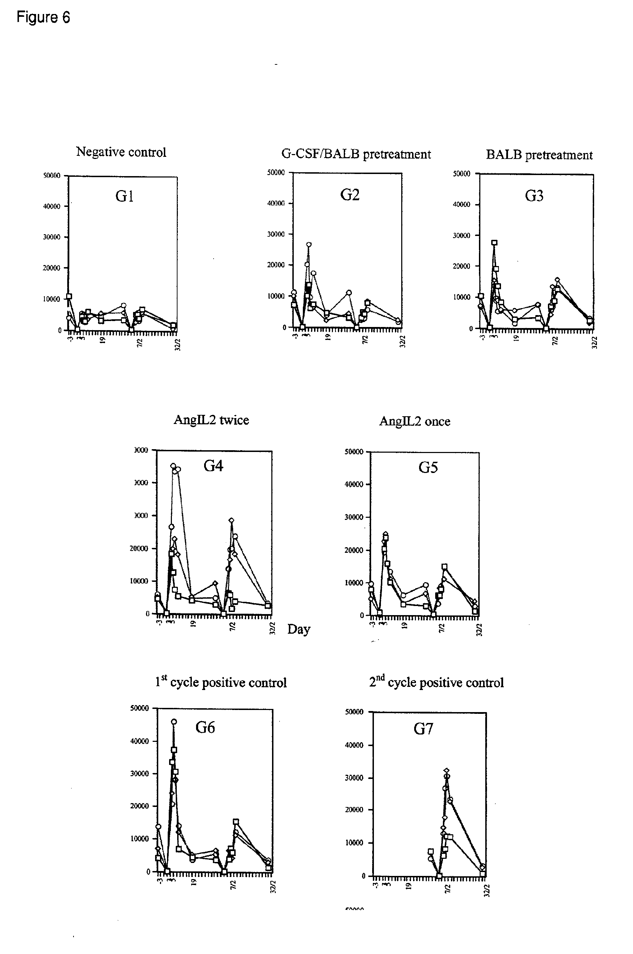

Fig. 6 (G1) shows that absolute white blood cell (WBC) counts in C57/BI6 were between 4000-10,000/µl prior to treatment. Between days 3 and 6 after injection of cyclophosphamide, nadir leukocyte level of 750-1500/µl were reached. In the first cycle, in group 4, 5 and 6, a rapid increase in absolute leukocyte counts was noticed starting from day 6. Control mice without any treatment in the first cycle (G1) only had a slow, steady rise in absolute WBC counts until day 10. Maximum values reached in these control groups were below 11,000/µl, whereas maximum counts reached in G5 and G6 were 49,000/µl. The other groups (G2 and G3) which received different pretreatment also demonstrated a slow rise in absolute leukocyte counts. Maximum values reached in these groups were below 30,000/µl. In the second cycle of chemotherapy, the absolute leukocyte counts reached 30,000/µl in the positive control group 7 which did not receive any treatment previously. In the other groups which received pretreatment (G2, G3 and G5) or the injection with hG-CSF secreting cells in the first cycle (G6), the absolute leukocyte counts only reached 10,000/µl. Only in group 4 which received twice injections with hG-CSF and AngIL2/BALB, the leukocyte counts still reached 25,000/µl in two of three mice. This is comparable to leukocyte counts reached by the positive control (G7) which never received injection with allogeneic cells. This protective effect on allogeneic hG-CSF secreting cells in the repetitive transplantation most likely results from administration of AngIL2 transfected fibroblasts.

## Claims

1. A polynucleotide comprising
a) a nucleotide sequence capable of acting as a eukaryotic promoter or a nucleotide sequence capable of acting as a viral promoter; and
b) a nucleotide sequence encoding a fusion protein which comprises a first polypeptide capable of binding to a cell surface receptor present on human cells and a second polypeptide which is toxic to a human cell after internalization of said second polypeptide by the human cell.

2. A polynucleotide according to claim 1 further comprising a nucleotide sequence capable of acting as a polyadenylation signal.

3. A polynucleotide according to claim 1 or 2 **characterized in that** the first polypeptide is capable of binding to a cell surface receptor which is present on human T cells.

4. A polynucleotide according to any of claims 1 to 3 **characterized in that** the first polypeptide is capable of binding to a cell surface receptor which is present on activated T cells but which is substantially not present on resting T cells and memory T cells.

5. A polynucleotide according to any of claims 1 to 4 **characterized in that** the first polypeptide is capable of binding to a cytokine receptor.

6. A polynucleotide according to any of claims 1 to 5 **characterized in that** the first polypeptide is capable of binding to IL-2 receptor.

7. A polynucleotide according to any of claims 1 to 6 **characterized in that** the first polypeptide is a cytokine.

8. A polynucleotide according to claim 7 **characterized in that** the cytokine is a human cytokine.

9. A polynucleotide according to any of claims 1 to 8 **characterized in that** the first polypeptide is IL-2 or a fragment thereof capable of binding to IL-2 receptor present on human cells.

10. A polynucleotide according to any of claims 1 to 9 **characterized in that** the first polypeptide is an antibody, an antibody fragment or a scFv fragment.

11. A polynucleotide according to any of claims 1 to 10 **characterized in that** the second polypeptide is not *Pseudomonas* exotoxin A or a fragment thereof.

12. A polynucleotide according to any of claims 1 to 11 **characterized in that** the second polypeptide is a ribonuclease or a fragment thereof having ribonuclease activity.

13. A polynucleotide according to any of claims 1 to 12 wherein the second polypeptide is angiogenin or a fragment thereof having ribonuclease activity.

14. A polynucleotide according to any of claims 1 to 13 further comprising a nucleotide sequence encoding a spacer consisting of 1 to 50 amino acids linking the first and the second polypeptide of the fusion protein.

15. A polynucleotide according to claim 14 wherein the spacer consists of 3 to 20 amino acids.

16. A polynucleotide according to claim 14 or 15 wherein the spacer consists of an amino acid sequence composed only of serine and glycine residues.

17. A eukaryotic cell transfected with a polynucleotide according to any of claims 1 to 16.

18. A eukaryotic cell according to claim 17 which is a human fibroblast cell.

19. A eukaryotic cell according to claim 18 which is a human skin fibroblast cell.

20. A eukaryotic cell according to any of claims 17 to 19 which is not derived from an immortalized cell line.

21. A pharmaceutical composition containing a eukaryotic cell according to any of claims 17 to 20.

22. A pharmaceutical composition according to claim 21 further containing a pharmaceutically acceptable diluent or carrier.

23. A method for the preparation of a pharmaceutical composition **characterized in that** a eukaryotic cell is transfected with a polynucleotide according to any of claims 1 to 16.

24. A method according to claim 23 wherein the eukaryotic cell is a human fibroblast cell.

25. A method according to claim 23 or 24 wherein the eukaryotic cell is a human skin fibroblast cell.

26. A method according to any of claims 23 to 25 **characterized in that** the eukaryotic cell is transfected using a liposomal transfection reagent.

27. A polypeptide comprising
a) IL-2 or a fragment thereof which is capable of binding to human IL-2 receptor;
b) optionally a spacer consisting of 1 to 50 amino acids; and
c) angiogenin or a fragment thereof which has ribonuclease activity.

28. A polypeptide according to claim 27 wherein said IL-2 is human IL-2 and/or said angiogenin is human angiogenin.

29. A polypeptide according to claim 27 or 28 wherein the spacer consists of 3 to 20 amino acids.

30. A polypeptide according to any of claims 27 to 29 wherein the spacer consists of an amino acid sequence composed only of serine and glycine residues.

31. A polynucleotide encoding a polypeptide according to any of claims 27 to 30.

32. The use of a polynucleotide according to any of claims 1 to 16 and 31 for the preparation of an immunosuppressive medicament.

33. The use of a eukaryotic cell according to any of claims 17 to 20 for the preparation of an immunosuppressive medicament.

34. The use of a polypeptide according to any of claims 27 to 30 for the preparation of an immunosuppressive medicament.


SEQUENCE LISTING

<110> CellGenix Technologie Transfer GmbH

<120> Selective cytotoxic fusion proteins

<130> IL2Ang

<140>
<141>

<160> 7

<170> PatentIn Ver. 2.1

<210> 1
<211> 15
<212> PRT
<213> Artificial Sequence

<220>
<223> Description of Artificial Sequence: Spacer

<400> 1
Gly Gly Gly Gly Ser Gly Gly Gly Gly Ser Gly Gly Gly Gly Ser
1               5                   10                  15

<210> 2
<211> 32
<212> DNA
<213> Artificial Sequence

<220>
<223> Description of Artificial Sequence: Primer

<400> 2
ggtgccacca tggtgatggg cctgggcgtt tt                                32

<210> 3
<211> 36
<212> DNA
<213> Artificial Sequence

<220>
<223> Description of Artificial Sequence: Primer

<400> 3
gctgccccct ccgcccggac gacggaaaat tgactg                                36

<210> 4
<211> 30
<212> DNA
<213> Artificial Sequence

<220>
<223> Description of Artificial Sequence: Primer

<400> 4
gatcaagctt accggtgcca ccatggtgat                                        30

<210> 5
<211> 40
<212> DNA
<213> Artificial Sequence

<220>
<223> Description of Artificial Sequence: Primer

<400> 5
cgccggatcc cccgcctccg ctgccccctc cgcccggacg                            40

<210> 6
<211> 46
<212> DNA
<213> Artificial Sequence

<220>
<223> Description of Artificial Sequence: Primer

<400> 6
cgggggatcc ggcggagggg gaagcgcacc tacttcaagt tctaca                     46

<210> 7
<211> 37
<212> DNA
<213> Artificial Sequence

<220>
<223> Description of Artificial Sequence: Primer

<400> 7
gatcggatcc agatcttcaa gttagtgttg agatgat                                37